# EUROPÄISCHE PATENTSCHRIFT

(11) **EP 3 100 691 B1**
(45) Veröffentlichungstag und Bekanntmachung des Hinweises auf die Patenterteilung: **27.11.2019**
(21) Anmeldenummer: 15170172.9
(22) Anmeldetag: 01.06.2015
(51) Int. Cl.: A61B 17/70

(54) **UNIPLANARES KNOCHENVERANKERUNGSELEMENT**
UNIPLANAR BONE ANCHORING ELEMENT
ÉLÉMENT D'ANCRAGE D'OS UNIPLANAIRE

(43) Veröffentlichungstag der Anmeldung: 07.12.2016
(73) Patentinhaber: Silony Medical International AG, 8500 Frauenfeld (CH)
(72) Erfinder: HEUER, Frank, 70794 Filderstadt (DE); WIRTH, Enrico, 78166 Donaueschingen (DE); TRAUTWEIN, Frank, 70794 Filderstadt (DE)
(74) Vertreter: Stolmár & Partner Patentanwälte PartG mbB

(56) Entgegenhaltungen:
- EP-A1- 1 923 011
- EP-A2- 2 198 793
- WO-A1-99/03415
- US-A1- 2013 023 941
- US-A1- 2014 121 703
- US-B1- 8 986 349

## Beschreibung

### Gebiet der Erfindung:

Die Erfindung betrifft ein uniplanares Knochenverankerungselement und insbesondere eine uniplanare Pedikelschraube, bei der der Schraubenschaft in nur einer Ebene schwenkbar ist.

### Stand der Technik

Im Stand der Technik sind verschiedene Knochenverankerungselemente wie beispielsweise Pedikelschrauben bekannt. Solche Knochenverankerungselemente werden beispielsweise dafür verwendet Wirbelfehlstellungen zu korrigieren, indem diese Verankerungselemente in den Wirbelknochen eingeschraubt werden und dann über Querstäbe miteinander verbunden werden, um die Fehlstellungen zu korrigieren und in der richtigen Position zu fixieren. Solche Verankerungselemente können als Monoaxialschrauben ausgebildet sein, bei der der Schraubenschaft fest mit dem Aufnahmeelement für den Querstab verbunden ist und bei der die Ausrichtung des Aufnahmeelements in Bezug auf die Längsachse des Schraubenschafts daher fixiert ist. Eine andere Ausgestaltung ist als Polyaxialschraube ausgebildet, bei der die Knochenschraube, die in den Wirbelknochen eingeschraubt wird, in Bezug auf das Aufnahmeelement beliebig verschwenkbar ist.

Es kann aber vorkommen, dass eine Verschwenkbarkeit in nur einer Ebene gewünscht ist. Dies ist beispielsweisebei Derotationsmanövern bei der Versorgung von skoliotischen Wirbelsäulen der Fall. Die EP 1 923 011 schlägt daher ein System vor, in dem zwei seitliche Durchgangsbohrungen in einem Aufnahmeelement angebracht sind, in die zwei Stifte eingesetzt werden können, gegen die dann zwei ebene Flächen am Schraubenkopf anstoßen. Durch die ebenen Flächen am Schraubenkopf wird im Zusammenspiel mit den beiden Stiften an den Seiten sichergestellt, dass der Schaft mit dem Knochengewinde sich lediglich in einer Ebene verschwenken lässt. Problematisch dabei ist jedoch, dass die Stifte lediglich mit einer abgerundeten Seite auf die ebenen Flächen der Schraube treffen und so eine sehr hohe Präzision beim Herstellen der Stifte und der Stiftbohrungen erforderlich ist. Denn selbst bei geringen Toleranzen kann der Stift den linienförmigen Kontakt mit der ebenen Fläche am Schraubenkopf verlieren und dadurch den linienförmigen Kontakt zwischen Stift und Fläche nicht aufrechterhalten, was zu einer Verschwenkbarkeit außerhalb der gewünschten Ebene führen kann. Ferner ist auch beim operativen Einsetzen des Knochenverankerungselements eine gewisse Vorsicht notwendig, damit die Stifte nicht aus den Durchgangsbohrungen des Verankerungselements herausfallen. US20130023941A offenbart ein Element nach dem Oberbegriff des Anspruchs 1.

### Darstellung der Erfindung

Aufgabe der vorliegenden Erfindung ist es daher ein uniplanares Knochenverankerungselement bereitzustellen, das einfach in der Herstellung und sicher in der Handhabbarkeit ist. Dies wird gewährleistet durch ein Knochenverankerungselement mit den Merkmalen des Anspruchs 1.

Ein erfindungsgemäßes uniplanares Knochenverankerungselement umfasst eine Verankerungsschraube mit einem Knochengewinde und einem Schraubenkopf, wobei der Schraubenkopf an zumindest einer Seite abgeflacht ist, ein Aufnahmeelement, wobei das Aufnahmeelement eine Durchgangsbohrung aufweist, die sich in einem unteren Bereich verjüngt und in der die Verankerungsschraube aufnehmbar ist, und zwei sich nach oben erstreckende Schenkel mit einem Gewindeprofil in einem oberen Bereich, das zum Anschrauben einer Befestigungsschraube ausgebildet ist, wobei das Aufnahmeelement im Sitzbereich es Verankerungsschraubenkopfes eine zumindest in Umfangsrichtung teilweise verlaufende Innennut aufweist und ein Lagerelement zum Einsetzen in die Innennut des Aufnahmeelements, wobei das Lagerelement an zumindest einer Seite teilweise abgeflacht ist, wobei die Nut und das Lagerelement derart ausgebildet sind, insbesondere komplementär, dass das Lagerelement in der Nut in einer vorbestimmten Lage positioniert ist. Die vorbestimmte Lage definiert nicht nur die Umfangsrichtung, die vorzugsweise durch die beiden Endwände der Nut festgelegt wird, sondern auch die Position der abgeflachten Seite des Lagerelements, die zum Schraubenkopf gerichtet sein soll. Dadurch wird gewährleistet, dass die abgeflachte Seite des Lagerelements an der abgeflachten Seite des Schraubenkopfes anliegt, da sich das Lagerelement in der Nut nicht verdrehen kann.

Vorzugsweise laufen die jeweiligen Enden in Umfangsrichtung aus. Das heißt, dass die Enden nicht abrupt aufhören, sondern der Nutgrund kontinuierlich ansteigt, bis die Nut nicht mehr vorhanden ist. Vorzugsweise weist dieser Nutauslauf eine Länge von höchstens von 7 mm, vorzugsweise 5mm und weiter vorzugsweise 3 mm. Der Auslauf ist bevorzugt mindestens 0,5 mm bis 1 mm lang. Ein solcher Auslauf hat den Vorteil, dass er einfach herzustellen ist, gleichzeitig das Lagerelement in der Nut aber durch den schon leichten Anstieg am Auslaufbeginn im zusammengebauten Zustand ein Verrutschen des Lagerelements in der Nut verhindert.

Die Innennut erstreckt sich in Umfangsrichtung, vorzugsweise über den halben Umfang, weiter vorzugsweise über dreiviertel des Umfangs. Die Umfangsnut und das Lagerelement sind jedoch zumindest halbkreisförmig umlaufend ausgebildet und haben den Vorteil, dass die Position des Lagerelements dann in jeder Richtung festgelegt ist. Sobald das Lagerelement in einer halbkreisförmigen Nut eingesetzt ist, kann es auch ohne in das Aufnahmeelement eingesetzte Schraube nicht mehr herausfallen.

Ferner kann in einer bevorzugten Ausführungsform der Querschnitt der Nut und das Lagerelement die vorbestimmte Lage definieren. Beispielsweise kann in der Nut eine senkrechte Fläche und an dem Lagerelement eine komplementäre zusätzliche senkrechte Fläche vorgesehen sein, so dass das Lagerelement in der Nut nur in einer Position sitzen kann. Ebenfalls möglich ist ein ovaler Querschnitt oder jede andere Form, die das Lagerelement in lediglich einer Position in die Nut einsetzbar macht. Bevorzugt ist der Nutgrund als ebene Fläche ausgebildet, so dass das Lagerlement mit einer entsprechenden Fläche sich am Nutgrund ausrichten kann. Die Innennut und insbesondere der Nutgrund kann bogenförmig in Umfangsrichtung ausgebildet sein, kann aber auch gerade verlaufen. Bei einem U-förmigen Lagerlement mit geraden Schenkeln können diese ein Verrutschen des Lagerelements in der Nut in Umfangsrichtung verhindern.

Die Innennut ist vorzugsweise unterhalb der Schenkel des Aufnahmeelements ausgebildet. Damit wird sichergestellt, dass die ebenen Anlageflächen des Schraubenkopfs und des Lagerelements nicht durch die Anordnung anderer Bauteile des Knochenverankerungselements beeinträchtigt werden.

Das Lagerelement ist U-förmig mit zwei im Wesentlichen parallelen Schenkeln ausgebildet, so dass der Schraubenkopf zwischen diesen parallelen Schenkeln aufgenommen werden kann. Ferner ist das Lagerelement vorzugsweise mit einem im Wesentlichen rechteckigen Querschnitt ausgebildet, was ein Anliegen der Flächen zwischen Nut und Lagerelement sowie zwischen Lagerelement und Schraubenkopf erleichtert. Die Kanten der Nut und des Lagerelements sind dabei aus bevorzugt mit Radien versehen, was den Kontakt und den festen Sitz zwischen den Einzelteilen aber nicht beeinträchtigt.

Ferner weist die Verankerungsschraube auf zwei im Wesentlichen gegenüberliegenden Seiten abgeflachte Stellen auf, so dass die Schraube auf beiden Seiten mit einem Lagerelement in Kontakt geraten kann. Insbesondere wird diese Ausführungsform mit dem U-förmigen Lagerelement verwendet.

### Kurze Beschreibung der Zeichnungen

Figur 1a zeigt eine isometrische Ansicht eines montierten uniplanaren Knochenverankerungselements gemäß der vorliegenden Erfindung;
Figur 1b zeigt eine Explosionsansicht des uniplanaren Knochenverankerungselements aus Figur 1a;
Figur 2a zeigt einen Längsschnitt eines montierten uniplanaren Knochenverankerungselements in einem weiteren Beispiel;
Figur 2b zeigt die Einzelteile eines montierten uniplanaren Knochenverankerungselements aus Figur 2a; und

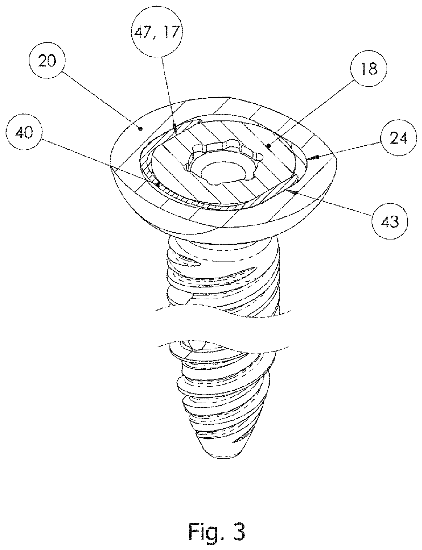

Figur 3 zeigt einen Schnitt durch das Aufnahmeelement am oberen Ende der Innenut sowie den Sitz eines U-förmigen Lagerelements auf dem Schraubenkopf.

### Beschreibung der bevorzugten Ausführungsformen

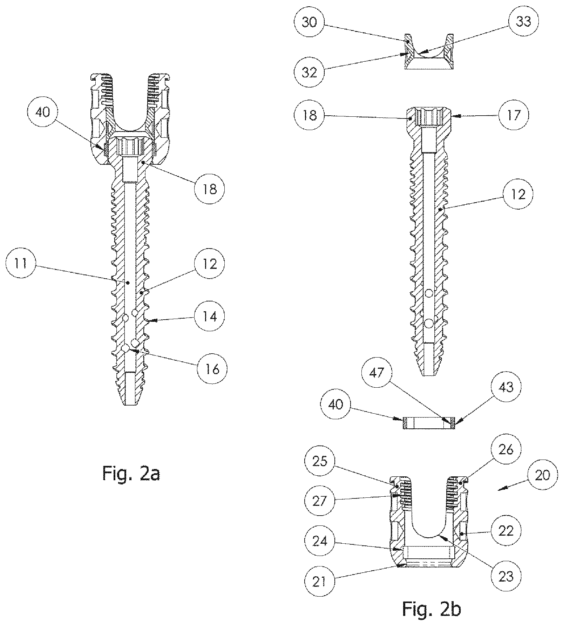

Wenn im Folgenden von oben und unten die Rede ist, dann wird dabei auf eine Anordnung Bezug genommen, die in Figur 2a zu sehen ist. Das obere Ende der Uniplanarschraube betrifft somit das obere Ende des Aufnahmeteils mit dem Gewinde, während das untere Ende die Spitze des Schraubenschafts betrifft. Ferner ist mit Axialrichtung die axiale Richtung des Knochenverankerungselements aus Figur 2a gemeint, die radiale Richtung steht senkrecht auf der axialen Richtung und eine Umfangsrichtung bezeichnet eine Richtung um die axiale Richtung herum.

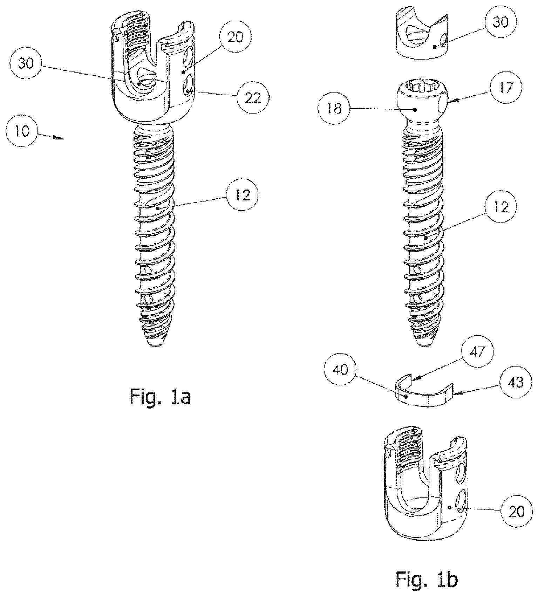

In Figur 1a ist eine isometrische Ansicht des erfindungsgemäßen Knochenverankerungselements 10 zu sehen. Vorliegend umfasst das Knochenverankerungselement 10 eine Schraube 12, die im Knochen verankert wird, ein Aufnahmeelement 20 zur Aufnahme der Schraube 12 und eines Querstabs (nicht gezeigt), ein Druckstück 30, mit den die Schraube 12 im Aufnahmeelement 20 montiert wird, und dem Lagerelement 40. Die Schraube 12 besteht aus einem Schraubenschaft und einem Schraubenkopf 18. Am Schraubenschaft ist ein Knochengewinde 14 angeordnet, das zumindest teilweise mehrgängig ausgebildet sein kann. Die Schraube kann eine Durchgangsbohrung 11 aufweisen, die im unteren Bereich Öffnungen 16 im Schraubenschaft umfassen kann. Mit diesen Löchern 16 kann eine in den Knochen eingedrehte Schraube mit Knochenzement im Knochen befestigt werden, in dem der Knochenzement durch die Bohrung an das untere Ende der Schraube 12 gebracht wird. Am Schraubenkopf 18 ist auf zumindest einer Seite ein abgeflachter Bereich 17 vorgesehen. bevorzugt und wie in den Figuren gezeigt ist dieser abgeflachte Bereich 17 auf zwei im Wesentlichen gegenüberliegenden Seiten des Schraubenkopfes 18 vorgesehen. Im Prinzip können auch mehr als zwei gegenüberliegende Abflachungen vorgesehen sein, beispielsweise vier abgeflachte Bereiche, die jeweils um 90° zueinander versetzt sind, oder auch acht, die dann jeweils um 45° zueinander versetzt sind. Eine solche Schraube hat mehrere Endstellungen, in der die abgeflachten Bereiche am Schraubenkopf mit den abgeflachten Bereichen am Lagerelement zusammenwirken können.

Das Druckstück 30, in dem das Aufnahmeelement 20 eingesetzt, dient dazu die Schraube im Aufnahmeelement 20 zu halten. Dazu wird bei montiertem Knochenverankerungselement, wie in Figur 1a zu sehen, in der Bohrung 22 eine Presskraft aufgebracht, die wiederum in die Bohrungen 32 des Druckstücks 30 eine Kraft aufbringen und so das Druckstück über der Schraube zusammendrücken. Damit kann die Schraube 12 zwar noch verschwenkt werden, aber nicht mehr aus dem Aufnahmeelement herausfallen, da der Bereich der Bohrung 22 in das Druckstück 30 hineingepresst wird. Das Druckstück 30 weist eine Vertiefung 33 auf, in der später der Querstab eingelegt wird.

Das Aufnahmeelement 20 weist eine Durchgangsbohrung mit einem sich verjüngenden unteren Ende 21 auf. In diesem sich verjüngenden unteren Ende 21 wird der untere Teil des Schraubenkopfes 18 gelagert. Das Aufnahmeelement 20 weist ferner die oben erwähnten Bohrungen 22 zum Zusammenpressen des Druckstück auf und zwei Schenkel 25, 26, die sich nach oben erstrecken und ein Innengewinde 27 umfassen. In das Innengewinde 27 wird später eine Druckschraube eingeschraubt, die auf den Querstab drückt und so alle Bauteile des Knochenverankerungselements fixiert. Vorliegend ist dieses Gewinde als Innengewinde gezeigt, es ist aber auch problemlos möglich, dies als Außengewinde auszuführen und keine Madenschraube als Druckschraube zu verwenden, sondern eine Schraubenmutter.

Das Aufnahmeelement 20 weist eine Aufnahme 23 für den Querstab auf, die als U-förmige Aussparung ausgebildet ist. In dieser Aussparung wird dann der Querstab eingelegt und die Befestigungsschrauben (hier eine Madenschraube) in das Gewinde 27 eingeschraubt. In einem unteren Bereich des Aufnahmeelements 20, vorzugsweise unterhalb der Schenkel 26, 25 ist eine Nut 24 vorgesehen, in die das Lagerelement 40 eingesetzt wird. Vorzugsweise weist die Nut ebenfalls ebene Flächen auf. Die Nut verläuft vorzugsweise umlaufend und entspricht im Wesentlichen der Gestalt des Lagerelements 40, sowohl in Umfangsrichtung, als auch im Querschnitt. Wenn das Lagerelement 40 als Ring oder teilkreisförmiger Ring ausgebildet ist, ist der Querschnitt der Nut unwichtig, da die Positionierung der Anschlagflächen 47 für die abgeflachten Bereiche 17 des Schraubenkopfes durch die Ringform in Position gehalten werden. Wenn das Lagerelement jedoch nicht zumindest halbkreisförmig ausgebildet ist, ist es insbesondere bei kürzeren Lagerelementen, die nur auf einer Seite des Aufnahmeelements eingesetzt werden, vorteilhaft, wenn die Positionierung der ebenen Anschlagfläche über den Querschnitt des Lagerelements und der Nut erfolgt. Dazu ist vorzugsweise ein im Wesentlichen quadratischer oder rechteckiger Querschnitt geeignet, es kann aber ebenso ein ovaler Querschnitt oder eine andere Form sein, die eine vorbestimmte Lage des Lagerelements 40 in der Nut bereitstellen können.

Das Lagerelement 40 ist im Beispiel der Figur 2 als Ring mit zumindest einem Schlitz oder teilweiser Ring ausgebildet, damit das Lagerelement 40 federnd zusammengedrückt werden kann und in die Nut des Aufnahmeelements 20 eingesetzt werden kann. Dies kann aber bspw. auch durch die Änderung des Materials oder der Materialeigenschaft (bspw. der Dicke) ermöglicht werden. So kann auch ein geschlossener Ring sich ausreichend zusammendrücken lassen, so dass er in die Innennut einsetzbar ist. Das Einsetzen kann von oben oder von unten erfolgen. Wenn das Lagerelement 40 ringförmig ausgebildet ist, kann es aus der umlaufenden Nut nicht mehr herausfallen.

Damit sich das Lagerelement 40 auch nicht in der Nut in Umfangsrichtung des Aufnahmeelements verdrehen kann, ist die Nut im Aufnahmeelement 20 vorzugsweise auch nicht vollumfänglich ausgeführt, sondern nur entsprechend den Maßen des Lagerelements 40. Hier weist vorzugsweise das Lagerelement 40 auch eine weitere ebene Fläche 43 auf, die mit dem ebenen Nutgrund der Nut 24 im Aufnahmeelement 20 zusammenwirkt.

Erfindungsgemäss ist das Lagerelement 40 U-förmig ausgebildet, so dass die beiden im Wesentlichen parallelen Schenkel des "U" ebenfalls ein Verdrehen des Lagerelements 40 in der Nut 24 verhindern können, falls die Nut 24 doch vollumfänglich im Aufnahmeelement 20 ausgebildet ist. Durch diese zwei im Wesentlichen parallelen Schenkel und deren senkrechte Außenflächen 43 lässt sich das Lagerelement 40 nicht mehr in der Nut 24 in Umfangsrichtung verdrehen.

Das Lagerelement 40 weist zumindest eine abgeflachte Seite 47 auf, die mit dem abgeflachten Bereich 17 des Schraubenkopfes 18 zusammenwirkt und ein Verschwenken in ausschließlich einer gewünschten Ebene der Knochenschraube sicherstellt. Dadurch dass das Lagerelement auch flächig an dem flächigen Bereich des Schraubenkopfes anliegt, ist auch bei kleinerer Ungenauigkeit ein Verschwenken in anderen Ebenen als der gewünschten nicht möglich.

## Patentansprüche

1. Uniplanares Knochenverankerungselement (10), umfassend
eine Verankerungsschraube (12) mit einem Knochengewinde (14) und einem Schraubenkopf (18), wobei der Schraubenkopf an zumindest einer Seite (17) abgeflacht ist;
ein Aufnahmeelement (20), wobei das Aufnahmeelement eine Durchgangsbohrung aufweist, die sich in einem unteren Bereich (21) verjüngt und in der die Verankerungsschraube (12) aufnehmbar ist, und zwei sich nach oben erstreckende Schenkel (25, 26) mit einem Gewindeprofil (27) in einem oberen Bereich, das zum Anschrauben einer Befestigungsschraube ausgebildet ist, wobei das Aufnahmeelement (20) im Sitzbereich des Verankerungsschraubenkopfes (18) eine zumindest in Umfangsrichtung teilweise verlaufende Innennut (24) aufweist; und
ein Lagerelement (40) zum Einsetzen in die Innennut (24) des Aufnahmelements (20), wobei das Lagerelement (40) an zumindest einer Seite (47) teilweise abgeflacht ist;
**dadurch gekennzeichnet, dass**
die Nut und das Lagerelement derart komplementär ausgebildet sind, dass das Lagerelement in der Nut in einer vorbestimmten Lage positioniert ist, wobei das Lagerelement (40) U-förmig mit zwei im Wesentlichen parallelen Schenkeln ausgebildet ist.

2. Knochenverankerungselement (10) nach Anspruch 1, bei dem die Enden der Innennut (24) in Umfangsrichtung auslaufen.

3. Knochenverankerungselement (10) nach einem der vorhergehenden Ansprüche, bei dem der Querschnitt der Nut (24) und des Lagerelements (40) die vorbestimmte Lage definieren.

4. Knochenverankerungselement (10) nach einem der vorhergehenden Ansprüche, bei dem die Innennut (24) unterhalb der Schenkel (25, 26) des Aufnahmeelements ausgebildet ist.

5. Knochenverankerungselement (10) nach einem der vorhergehenden Ansprüche, bei dem das Lagerelement (40) mit einem im Wesentlichen rechteckigen Querschnitt ausgebildet ist.

6. Knochenverankerungselement (10) nach einem der vorhergehenden Ansprüche, bei dem die Verankerungsschraube (12) auf zwei im Wesentlichen gegenüberliegenden Seiten abgeflacht ist.

## Claims

1. Uniplanar bone anchoring element (10), comprising
an anchoring screw (12) with a bone thread (14) and a screw head (18), wherein the screw head is flattened on at least one side (17);
a receiving element (20), wherein the receiving element has a through-bore which tapers in a lower region (21) and in which the anchoring screw (12) can be received, and two limbs (25, 26) which extend upwards and have a threaded profile (27) in an upper region, which is designed for screwing on a securing screw, wherein the receiving element (20) has an inner groove (24) which runs at least partly in the circumferential direction in the seat region of the anchoring screw head (18); and
a bearing element (40) for inserting into the inner groove (24) of the receiving element (20), wherein the bearing element (40) is partly flattened on at least one side (47);
**characterized in that**
the groove and the bearing element are designed in a complementary manner such that the bearing element (40) is positioned in the groove in a predetermined position and the bearing element (40) is designed U-shaped with two substantially parallel limbs.

2. Bone anchoring element (10) according to claim 1, wherein the ends of the inner groove (24) taper off in the circumferential direction.

3. Bone anchoring element (10) according to any one of the preceding claims, wherein the cross sections of the groove (24) and of the bearing element (40) define the predetermined position.

4. Bone anchoring element (10) according to any one of the preceding claims, wherein the inner groove (24) is formed below the limbs (25, 26) of the receiving element.

5. Bone anchoring element (10) according to any one of the preceding claims, wherein the bearing element (40) is designed with a substantially rectangular cross section.

6. Bone anchoring element (10) according to any one of the preceding claims, wherein the anchoring screw (12) is flattened on two substantially opposite sides.

## Revendications

1. Elément d'ancrage d'os uniplanaire (10), comprenant
une vis d'ancrage (12) présentant un filetage à pénétration dans l'os (14) et une tête de vis (18), la tête de vis étant aplatie sur au moins un côté (17) ;
un élément de réception (20), l'élément de réception présentant un trou débouchant, lequel est conique dans une région inférieure (21) et dans lequel la vis d'ancrage (12) peut être reçue, et deux bras (25, 26) s'étendant vers le haut avec un profil de vis (27) dans une région supérieure, lequel est formé pour être vissé sur une vis de fixation, dans lequel, dans la région d'assise de la tête de vis d'ancrage (18), l'élément de réception (20) présente une rainure interne (24) s'étendant partiellement au moins dans le sens circonférentiel ; et
un élément porteur (40) pour l'insertion dans la rainure interne (24) de l'élément de réception (20), l'élément porteur (40) étant partiellement aplati sur au moins un côté (47) ;
**caractérisé en ce que**
la rainure et l'élément porteur sont formés de manière complémentaire de telle sorte que l'élément porteur soit positionné dans la rainure dans une position prédéterminée, l'élément porteur (40) ayant sensiblement la forme d'un U à deux bras sensiblement parallèles.

2. Elément d'ancrage d'os (10) selon la revendication 1, dans lequel les extrémités de la rainure interne (24) s'amincissent dans le sens circonférentiel.

3. Elément d'ancrage d'os (10) selon l'une des revendications précédentes, dans lequel les coupes transversales de la rainure (24) et de l'élément porteur (40) définissent la position prédéterminée.

4. Elément d'ancrage d'os (10) selon l'une des revendications précédentes, dans lequel la rainure interne (24) est formée en dessous des bras (25, 26) de l'élément de réception.

5. Elément d'ancrage d'os (10) selon l'une des revendications précédentes, dans lequel l'élément porteur (40) est formé avec une coupe transversale sensiblement rectangulaire.

6. Elément d'ancrage d'os (10) selon l'une des revendications précédentes, dans lequel la vis d'ancrage (12) est aplatie sur deux côtés sensiblement opposés.
